(19) Europäisches Patentamt
European Patent Office
Office européen des brevets

(11) Publication number: **0 281 951 B1**

(12) **EUROPEAN PATENT SPECIFICATION**

(45) Date of publication of patent specification: **15.06.94**

(51) Int. Cl.5: **C08B 37/00**, C08B 31/00, C07B 57/00

(21) Application number: **88103318.7**

(22) Date of filing: **03.03.88**

Divisional application 93105963.8 filed on 03/03/88.

The file contains technical information submitted after the application was filed and not included in this specification

# (54) Alkyl-phenylcarbamate derivative of polysaccharide.

(30) Priority: **04.03.87 JP 49144/87**
**20.03.87 JP 65989/87**
**08.02.88 JP 26995/88**

(43) Date of publication of application:
**14.09.88 Bulletin 88/37**

(45) Publication of the grant of the patent:
**15.06.94 Bulletin 94/24**

(84) Designated Contracting States:
**BE CH DE FR GB IT LI NL**

(56) References cited:
**EP-A- 0 157 365**
**EP-A- 0 238 044**
**FR-A- 2 101 630**

**"MODIFICATIONS OF POLYMERS", 1980, ed. C.E. Carraer et al., pages 371-380, American Chemical Society, Washington, US; C.L. McCORMICK et al.: "Homogeneous solution reactions of cellulose, chitin, and other polysaccharides"**

(73) Proprietor: **DAICEL CHEMICAL INDUSTRIES, LTD.**
**No. 1-Banchi, Teppo-cho**
**Sakai-shi Osaka-fu 590(JP)**

(72) Inventor: **Oakamoto, Yoshio**
**24-11, Mukonoshohigasi 1-chome**
**Amagasaki-shi Hyogo(JP)**
Inventor: **Hatada, Koichi**
**4-11, Asahioka 3-chome**
**Ikeda-shi Osaka(JP)**

(74) Representative: **Patentanwälte Grünecker, Kinkeldey, Stockmair & Partner**
**Maximilianstrasse 58**
**D-80538 München (DE)**

**EP 0 281 951 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF CHROMATOGRAPHY, vol. 363, no. 2, 29th August 1986, Elsevier Science Publishers B.V., Amsterdam, NL; Y. OKAMOTO et al.: "Chromatographic resolution. X1*. Controlled chiral recognition of cellulose triphenylcarbamate derivatives supported on silica gel"

"Advanced Organic Chemistry, 1977, pages 146 to 150".

## Description

The present invention relates to an optically active alkylphenylcarbamate derivative of a polysaccharide, which is suitable as a separating agent for separating optical isomers from a racemic mixture.

It is known that a packing for a liquid chromatographic column comprising cellulose trisphenylcarbamate as the stationary phase has an excellent optical resolving capacity (Okamoto, Hatakeda et al., Journal of the American Chemical Society, 106, 5357 (1984)).

Modifications of Polymers, American Chemical Society, 1980, p. 371-380, discloses carbamate derivatives of cellulose and amylose wherein the carbamoyl group is p-methylphenyl substituted.

Journal of Chromatography, vol. 363, no. 2, 29th August 1986, p. 174, discloses 3,4-$(CH_3)_2$, 3,5-$(CH_3)_2$ and 2,6-$(CH_3)_2$ substituted cellulose triphenylcarbamate.

EP-A-238044 which is prior art according to Art. 54(3) EPC discloses alkyl-substituted phenylcarbamate derivatives of a polysaccharide exclusive of cellulose.

EP-A-157367 discloses a method of separating chemical substances by treatment with a carbamate derivative of cellulose or amylose except an aromatic carbamate of cellulose.

It is the object of the present invention to provide a compound having an excellent chiral discriminating power.

Said object is achieved by an optically active derivative of a polysaccharide wherein 80 to 100 percent of hydrogen in the hydroxyl groups of which have been reacted to form an alkylphenylcarbamoyl group, said derivative being cellulose-tris-(4-t-butylphenylcarbamate) or amylose-tris(4-t-butylphenylcarbamate).

The carbamoyl group of these compounds has the following formula:

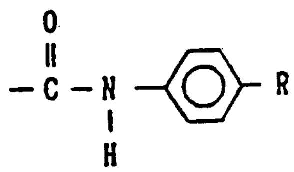

wherein R is t-butyl.

The cellulose type carbamate derivative comprises repeating constitutional units represented by the following general formula:

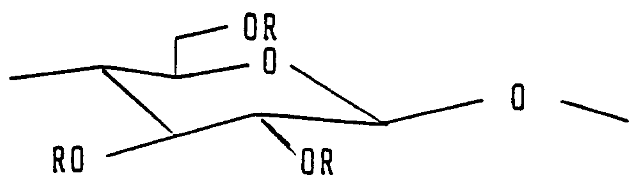

wherein 80 to 100 % of R's are substituted by the carbamoyl group.

The invention moreover provides a separating agent comprising the alkylphenylcarbamate derivative of the invention. It may further comprise a carrier. The agent serves to obtain from a racemic mixture of optical isomers, a pure optical isomer or a mixture of optical isomers having a higher enantiomeric content than the starting mixture by bringing the mixture into contact therewith.

The invention also provides an optical resolution method or an optical separation method with an unexpected improvement in view of the capacity factor, the separation factor and the resolution.

Any of synthetic, natural and modified natural cellulose or amylose which are optically active can be used as the polysaccharide in the present invention, but a polysaccharide having a high regularity in the bonding manner is preferred. For example, there can be mentioned α-1,4-glucan (amylose and amylopectin) and starch containing amylose.

The number-average degree of polymerization (the average number of pyranose or furanose rings contained in one molucule) ranges from 2 to 1,000; it is preferably not less than 5, more preferably not less

than 10. In view of the easiness of handling, it is preferred that it is not larger than 500.

The alkylphenylcarbamate of a polysaccharide according to the invention may have terminal groups of hydrogen or a usual group of the polysaccharide.

The reaction ordinarily adopted for forming a urethane from an alcohol and an isocyanate can be directly applied to the synthesis of the carbamate derivative of the present invention. For example, the carbamate derivative of the present invention can be synthesized by reacting a corresponding polysaccharide with a corresponding isocyanate in an appropriate solvent in the presence of a Lewis base such as a tertiary amine or a Lewis acid such as a tin compound as the catalyst. Furthermore, the isocyanate can be easily synthesized by reacting an amino group of a corresponding aniline derivative with phosgene.

When the polysaccharide carbamate derivative of the present invention is used as a separating agent for separating a compound or its optical isomer, there may be generally adopted chromatographic methods such as gas chromatography, liquid chromatography and thin-layer chromatography. Furthermore, the polysaccharide carbamate derivative of the present invention can be applied to a separation method using a membrane.

When the polysaccharide carbamate derivative of the present invention is used for liquid chromatography, the derivative is generally packed into a column in a powdery form, and it is preferred that the derivative is pulverized or formed into beads and that the obtained particles are porous. In order to improve the pressure resistance of the separating agent, prevent swelling or shrinkage caused by solvent substitution and an increase of the number of theoretical plates, it is preferred that the polysaccharide be supported on a carrier.

When the polysaccharide is used as a powder, it is preferred that the particle size or carrier size is 1 µm to 1 mm, especially 1 to 300 µm, though the preferred size differs to some extent according to the size of the column. It is preferred that the carrier is porous, and that the average pore size is 1 nm (10 Å) to 100 µm, preferably 5 to 5000 nm (50 to 50000 Å). The amount of the polysaccharide carbamate derivative supported on the carrier is 1 to 100 % by weight, preferably 5 to 50 % by weight, based on the carrier.

Either a chemical method or a physical method can be adopted for supporting the polysaccharide carbamate derivative on the carrier. As a physical method, there can be mentioned a method in which the polysaccharide carbamate derivative is dissolved in a solvent capable of dissolving the derivative therein, the solution is sufficiently mixed with the carrier and the solvent is distilled under reduced pressure or heating or in an air flow, and a method in which the polysaccharide carbamate derivative is dissolved in a solvent capable of dissolving the derivative therein, the solution is sufficiently mixed with the carrier and the mixture is dispersed in a solvent incapable of dissolving the derivative therein to diffuse the former solvent. The obtained separating agent is subjected to an appropriate post-treatment such as heating, addition of a solvent or washing to increase the separating capacity.

A porous organic carrier or a porous inorganic carrier can be used as the carrier. A porous inorganic carrier is preferred. As suitable examples of the porous organic carrier there can be mentioned polystyrene, polyacrylamide and polyacrylate, and as suitable examples of the porous inorganic carrier, there can be mentioned silica, alumina, magnesia, glass, kaolin, titanium oxide and silicate. The surface of the carrier may be treated to improve the affinity with the polysaccharide carbamate derivative or the surface characteristics of the carrier. As a surface treatment method, there can be mentioned a silane treatment using an organic silane compound and a plasma polymerization surface treatment.

The kind of developing solvent for liquid chromatography or thin-layer chromatography is not particularly critical, as far as it neither dissolves the polysaccharide carbamate derivative nor reacts therewith. When the polysaccharide derivative is chemically bonded with the carrier or is insolubilized by crosslinking, any developing solvent can be used, as far as it does not react with the polysaccharide carbamate derivative.

In case of thin-layer chromatography, a layer of the separating agent composed of particles having a size of 0.1 µm to 0.1 mm and, if necessary, a small amount of a binder, which has a thickness of 0.1 to 100 mm, is formed on a supporting plate.

In the membrane separation method, the separating agent is formed into a hollow yarn or film and is used in this form.

The polysaccharide carbamate derivative of the present invention is especially effective for separation of various compounds. It is particularly effective for separation of optical isomers, which is difficult according to conventional techniques, that is, as a packing for the optical resolution.

The present invention is further illustrated by the following examples.

The definitions of the terms used in the examples are as follows:

$$\text{Capacity factor } (k') = \frac{\left(\text{(retention time of enantiomer)} - \text{(dead time)}\right)}{\text{(dead time)}}$$

$$\text{Separation factor } (\alpha) = \frac{\text{capacity factor of more strongly adsorbed enantiomer}}{\text{capacity factor of more weakly adsorbed enantiomer}}$$

$$\text{Resolution (Rs)} = \frac{2\text{x(distance between peaks of more strongly adsorbed and more weakly adsorbed enantiomers)}}{\text{sum of band widths of both peaks}}$$

Example 1

Cellulose tris(4-t-butylphenylcarbamate) was synthesized as follows:

(1) Isocyanating 4-t-butylaniline

While carbon tetrachloride was refluxed, fuming sulfuric acid was added dropwise to evolve phosgene. When the atmosphere in the apparatus was sufficiently replaced by phosgene, a 4-t-butylaniline toluene solution (containing 6.00 g of 4-t-butylaniline in 250 ml of toluene) was added dropwise and the temperature was gradually elevated to allow the reaction to proceed while the toluene was refluxed. Yield: 5.61 g (79.6 %).

Then the toluene was removed by atmospheric distillation and the isocyanate was distilled at 83.2°C/800 Pa (6 mmHg).

(2) Synthesizing cellulose tris(4-t-butylphenylcarbamate)

0,59 g of cellulose was stirred in about 80 ml of pyridine at 100°C for 1 hour. After 30 ml of the pyridine were distilled off, 2.72 g of 4-t-butylphenyl isocyanate were added, and the mixture was reacted at 100°C for 19 hours.

The contents were poured into methanol to cause precipitation, and the precipitate was centrifugally separated and dried at 60°C for 2 hours by keeping the temperature constant. Yield: 1.56 g (67.5 %).

Example 2

Amylose tris(4-t-butylphenylcarbamate) was synthesized as follows:
0.51 g of amylose were stirred in about 75 ml of pyridine at 100°C for 2 hours. Then about 30 ml of the pyridine were distilled off, and after 2.89 g of 4-t-butylphenylisocyanate were added, the reaction was carried out for 20 hours.

The contents were poured into methanol to cause precipitation, and the precipitate was filtered through a glass filter, and was dried at 60°C for 2 hours by keeping the temperature constant. Yield: 1.18 g (55.7 %).

The analysis of the compounds obtained in Examples 1 and 2 is shown below.

| | Elemental analysis (%) | | |
|---|---|---|---|
| | C | H | N |
| Cellulose tris(4-t-butylphenylcarbamate) | 67.09 (68.10) | 7.07 (7.18) | 6.01 (6.11) |
| Amylose tris(4-t-butylphenylcarbamate) | 67.36 (68.10) | 7.20 (7.18) | 5.94 (6.11) |
| Figures in parenthesis are theroetical values. | | | |

Application Examples 1 und 2

Using the compounds prepared in Examples 1 and 2, separating agents and separating columns were prepared as shown below:

(1) Separating agents

Silica gel (YRK25) having a particle diameter of 7 µm and a pore diameter of 400 nm (4000 Å) whose surfaces have been treated with 3-aminopropyltriethoxysilane was used. About 0.75 g of cellulose tris(4-t-butylphenylcarbamate) and about 0,75 g of amylose tris(4-t-butylphenylcarbamate) were each dissolved in about 10 ml of tetrahydrofuran. Every time when about 3 g of YRK25 were used, about 2.5 ml of each of the tetrahydrofuran solutions was added, followed by shaking well. After the silica gel was uniformly wet, evaporation and vacuum drying (60°C) were effected. This procedure was repeated to support the whole of each compound.

(2) Separating columns

Each of the separating agents prepared in step (1) was classified by using hexane/2-propanol (90:10), and loaded into a stainless steel column having a length of 25 cm and an inner diameter of 0.46 cm by the slurry packing method. Mixtures of optical isomers shown in Table 6 were separated by using the thus produced separating columns. The separating conditions and the results are shown in Table 1.

## Table 1

| racemate | Example 1 | | | Example 2 | | |
|---|---|---|---|---|---|---|
| | $k_1'$ | α | $R_s$ | $k_1'$ | α | $R_s$ |
| 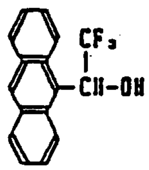 | 1.18(-) | 1.75 | 3.70 | 0.93(-) | 1.12 | |
| 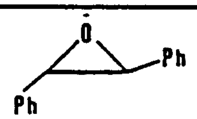 | 0.45(+) | 1.27 | 0.84 | 0.59(-) | ~1 | |
| Tröger base | 1.07(-) | 1.74 | 3.09 | 1.34(+) | 1.32 | 1.64 |
| $Co(acac)_3$ | 0.33(+) | 2.50 | 3.51 | 0.48(-) | ~1 | |
| 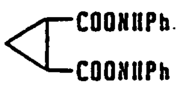 | 1.26(-) | 2.24 | 4.37 | 0.93(+) | 1.16 | |
| 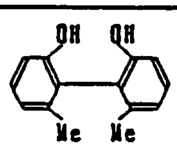 | 1.55(-) | 1.50 | 2.47 | 1.59(-) | 1.08 | |

**Table 1 (continued)**

| racemate | Example 1 $k_1'$ | Example 1 $\alpha$ | Example 1 $R_s$ | Example 2 $k_1'$ | Example 2 $\alpha$ | Example 2 $R_s$ |
|---|---|---|---|---|---|---|
| 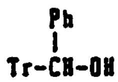 | 0.79(+) | 1.36 | 1.76 | 1.16(+) | 1.14 | 0.88 |
| 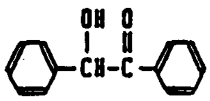 | 2.03(-) | 1.08 | | 3.67(-) | 1.17 | 1.69 |
| 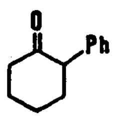 | 1.13(-) | 1.22 | 1.42 | 1.39(+) | 1.43 | 2.40 |
| 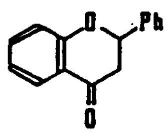 | 1.40(+) | 1.45 | 2.56 | 1.71 | 1.00 | |
| 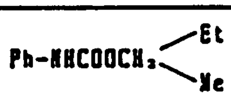 | 2.14(+) | 1.29 | 1.03 | 0.98 | 1.00 | |
| 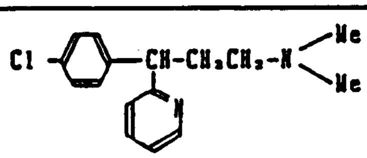 | 0.80(-) | 1.12 | | 1.13(-) | 1.45 | 1.36 |

**Table 1 (continued)**

| racemate | Example 1 | | | Example 2 | | |
|---|---|---|---|---|---|---|
| | $k_1'$ | $\alpha$ | $R_s$ | $k_1'$ | $\alpha$ | $R_s$ |
| EEDQ | 0.73(-) | 1.25* | | 0.35(-) | 1.22 | |
| 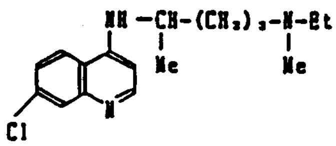 | 3.89(-) | 1.16 | | | | |
| Nicardipine | 8.04(-) | 1.21 | 1.29 | 3.42 | 1.00 | |
| 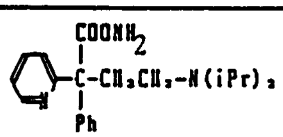 | 3.36(-) | 1.04 | | | | |

Hexane/2-propanol (90:10); 0.5 ml/min; 25°C

* Hexane/2-propanol (98:2)

## Claims

1. An optically active alkylphenylcarbamate derivative of a polysaccharide wherein 80 to 100 percent of hydrogen in the hydroxyl groups of which have been reacted to form the alkylphenylcarbamoyl group, said derivative being cellulose-tris(4-t-butylphenylcarbamate)or amylose-tris(4-t-butylphenylcarbamate).

2. The derivative of claim 1, which has a number-average polymerization degree of 2 to 1,000.

3. The derivative of claim 1, which is in the form of porous particles having a particle size of 1 µm to 1 mm and an average pore size of 1 nm (10A) to 100 µm.

4. A separating agent which comprises a carrier and 1 to 100 percent by weight, based on the carrier, of the derivative as defined in claim 1.

5. A method for obtaining from a racemic mixture of optical isomers a pure optical isomer or a mixture of optical isomers having a higher enantiomeric content than the starting mixture by bringing the mixture into contact with the derivative as defined in claim 1 or the separating agent as defined in claim 4.

6. The method of claim 5, which is conducted by means of chromatography.

**Patentansprüche**

1. Optisch aktives Alkylphenylcarbamatderivat eines Polysaccharids, worin 80 bis 100 Prozent des Wasserstoffs in seinen Hydroxylgruppen umgesetzt worden sind, um die Alkylphenylcarbamoylgruppe zu bilden, wobei das Derivat Cellulose-tris(4-t-butylphenylcarbamat) oder Amylose-tris-(4-t-butylphenylcarbamat) ist.

2. Derivat nach Anspruch 1, welches einen zahlendurchschnittlichen Polymerisationsgrad von 2 bis 1000 hat.

3. Derivat nach Anspruch 1, welches in Form von porösen Teilchen mit einer Teilchengröße von 1 µm bis 1 mm und einer durchschnittlichen Porengröße von 1 nm (10Å) bis 100 µm vorliegt.

4. Trennmittel, welches einen Träger und 1 bis 100 Gewichtsprozent, bezogen auf den Träger, des Derivats nach Anspruch 1 umfaßt.

5. Verfahren zum Erhalten eines reinen optischen Isomers oder eines Gemischs von optischen Isomeren mit einem höheren Enantiomerengehalt als das Ausgangsgemisch aus einem racemischen Gemisch von optischen Isomeren durch In-Berührung-Bringen des Gemischs mit dem Derivat nach Anspruch 1 oder dem Trennmittel nach Anspruch 4.

6. Verfahren nach Anspruch 5, welches mittels Chromatographie durchgeführt wird.

**Revendications**

1. Alkylphénylcarbamate dérivé optiquement actif de polysaccharide, dans lequel de 80 à 100 % des atomes d'hydrogène dans les groupes hydroxyle de celui-ci, ont réagi pour former le groupe alkylphénylcarbamoyle, ce dérive étant le tris-(4-t-butylphénylcarbamate) de cellulose ou le tris(4-t-butylphénylcarbamate) d'amylose.

2. Dérivé selon la revendication 1, ayant un degré de polymérisation moyen en nombre de 2 à 1 000.

3. Dérivé selon la revendication 1, sous la forme de particules poreuses ayant une taille particulaire de 1 µm à 1 mm, et une taille moyenne de pore de 1 nm (10 Å) à 100 µm.

4. Agent de séparation, comprenant un support, et de 1 à 100 % en poids par rapport au support, du dérivé selon la revendication 1.

5. Procédé d'obtention à partir d'un mélange racémique d'isomères optiques, d'un isomère optique pur ou d'un mélange d'isomères optiques ayant une teneur en énantiomère supérieure à celle du mélange de départ, selon lequel on met en contact le mélange avec le dérivé selon la revendication 1, ou l'agent de séparation selon la revendication 4.

6. Procédé selon la revendication 5, mis en oeuvre par chromatographie.

Fig. 1

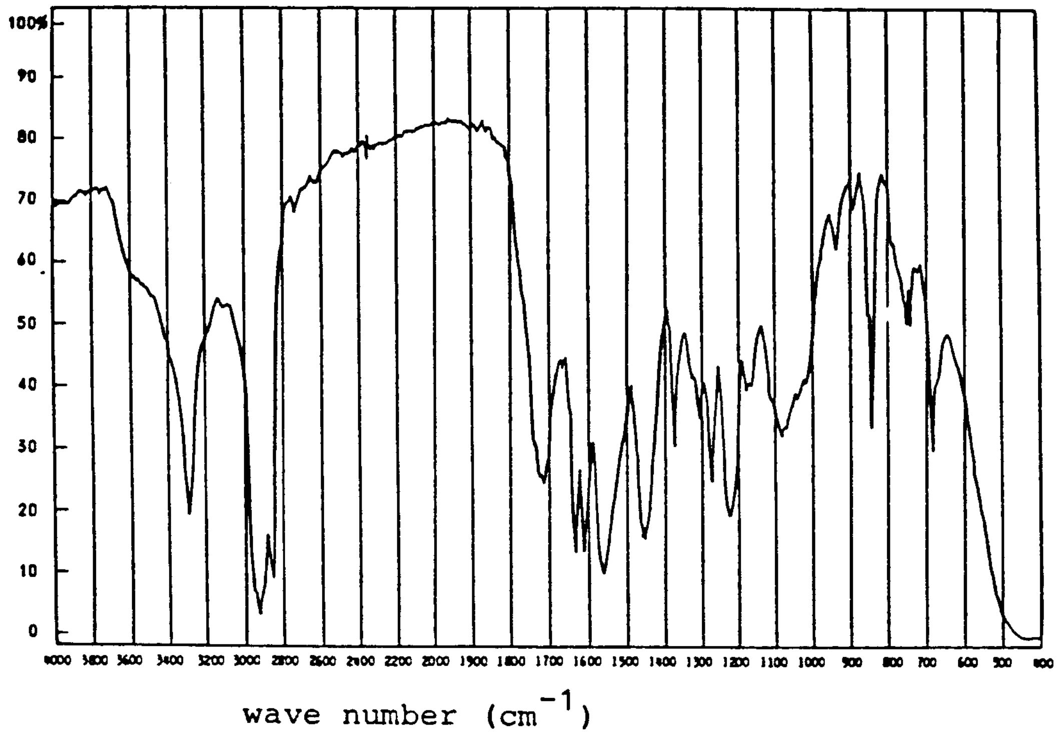
100%
90
80
70
60
50
40
30
20
10
0
wave number (cm$^{-1}$)

Fig. 2

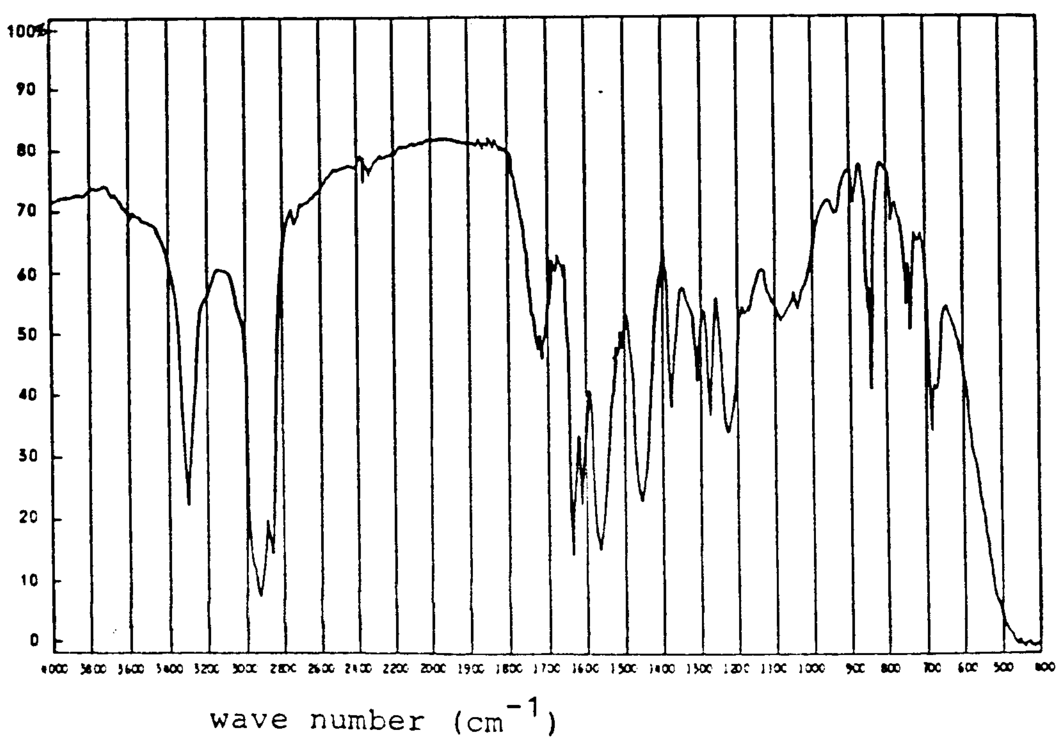
100%
90
80
70
60
50
40
30
20
10
0
wave number (cm$^{-1}$)